# EUROPEAN PATENT APPLICATION

(11) **EP 1 332 719 A1**
(43) Date of publication of application: **06.08.2003**
(21) Application number: 03075200.0
(22) Date of filing: 20.01.2003
(51) Int. Cl.: A61B 5/05, G01V 3/10

(54) **Method and apparatus to detect metal fragments in patients**

(30) Priority: 23.01.2002 GB 0201465; 14.05.2002 GB 0210971
(71) Applicant: Roke Manor Research Limited, Romsey, Hampshire SO51 0ZN (GB)
(72) Inventor: Richardson, Christopher Kieth, Romsey, Hants SO51 6EX (GB)
(74) Representative: Neill, Andrew Peter

(57) **Abstract**

A detector to detect metal fragments (especially in pre MRI scanning) comprising: a first loop of conducting material, means to feed an rf signal to the first loop, a second loop of conducting material, and means to detect an induced rf signal consequent to said rf signal in said first loop, said second loop being geometrically symmetrically arranged with said first loop such that the rf signal induced is therein is substantially zero when there are no metal fragments in the close proximity of the sensor. Preferably the second loop comprises two halves arranged in anti-phase, such in the form of a figure of eight. The loops are arranged to be substantially co-planar, such as on a PCB.

## Description

This invention relates to a method and apparatus of detecting metal fragments in patients, particularly pre-MRI patients.

If there are ferromagnetic fragments located in patients and these are undetected prior to undergoing a MRI scan, the scanner's magnetic field will cause the fragment to move, causing injury. The problem is generally not with medical implants as these are known to the patient and doctor, but it is with unsuspected objects such as shrapnel or splinters in people who have previously worked with metal.

The current method of determining is to CT-scan the patients who are suspected of having metal fragments. The problems result in delays to the MRI scan schedule. Further the CT method cannot distinguish between materials. Another problem is that it means the patents are exposed to X-rays. As a consequence radiologists have to err on the side of caution and assume that all metal objects detected are ferromagnetic so denying MRI scanning to many patients.

It is an object of the invention to overcome these problems and provide a device and method to detect small subcutaneous objects so as to reduce injury during MRI scanning.

The invention comprises a detector to detect metal fragments comprising: a first loop of conducting material, means to feed an rf signal to the first loop, a second loop of conducting material, and means to detect an induced rf signal consequent to said rf signal in said first loop, said second loop being geometrically symmetrically arranged with said first loop such that the rf signal induced is therein is substantially zero when there are no metal fragments in the close proximity of the sensor.

Preferably the second loop comprises two halves arranged in anti-phase, such in the form of a figure of eight. The loops are arranged to be substantially co-planar, such as on a PCB.

The invention will now be described with by means of example only.

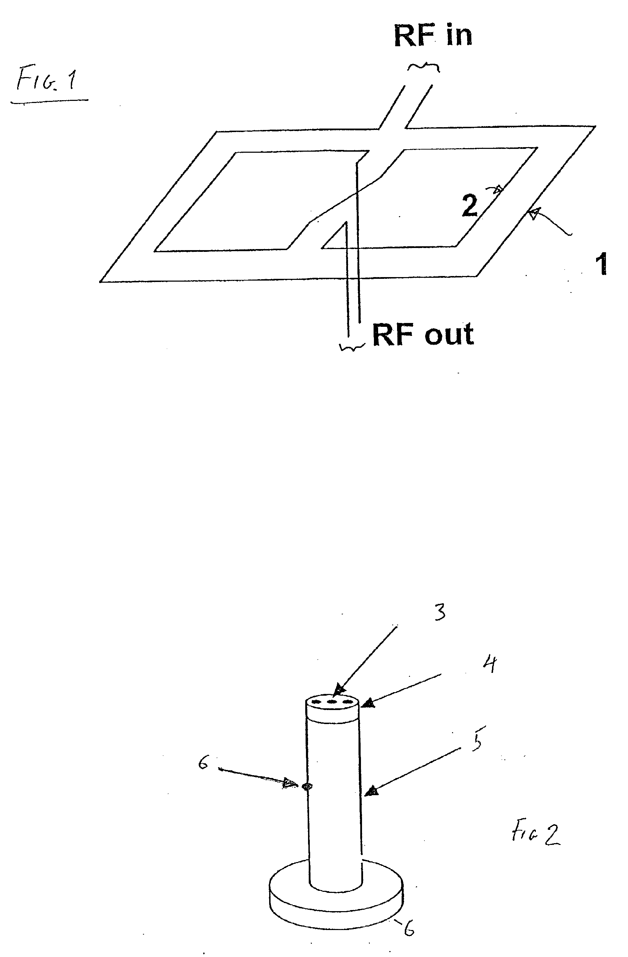

Figure 1 shows a schematic representation of one embodiment of the invention. The figure shows a first copper loop 1 and a second copper loop 2. These are laid out flat on a printed circuit board (not shown). The second loop is in the form of a figure of "8". In operation a very low power radio frequency is applied to one loop. The signal consequently induced in the second (figure of eight) loop cancels the and so no signal is detected in the output signal (the two halves of the loop are connected in anti-phase)

If the antenna system and the electrical characteristics of the material underneath are symmetrical, the radio frequency signal fed to the first loop produces no resultant signal from the figure of eight loop. If however there is a metal fragment under one half (side) of the figure of eight loop that will effect its rf characteristics relative to that of the other half of the loop, the system is then unbalanced and a net signal is produced. This is detected, amplified and signalled to an operator. The level of the signal peaks either side of a fragment when the antenna is moved from side to side over the area to be scanned. The sharp null in the middle of the scan can be used to accurately locate the object even if the antenna is very much larger than the object.

In a refined embodiment of the invention, the detector can also discriminate between ferromagnetic and non-ferromagnetic materials by measuring the phase of the detected signal. Ferromagnetic materials produce an output signal which is in phase with the energising waveform, whereas the output from ferromagnetic materials is at phase-quadrature with the energising signal.

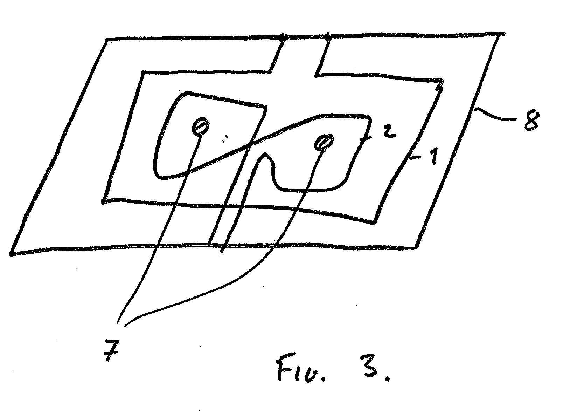

Figure 2 shows a schematic representation of a deployable device according to the invention in the form of a small hand held version and includes LED indicators 3, a lid 4 enclosing electronics and battery space 5 and search head 6 which comprises a PCP with the loops on it. An on-off button is 6 also provided. Detection of a metal fragment is indicated by LED's. which would also indicate ferrous or non-ferrous material. Alternatively an audible tone may be used (or in addition to) the LED's. A detector such as this can used to scan e.g. the patients eyes, where the danger of metallic fragments is the greatest, and detect fragments smaller than 0.1 cu mm.

Such a device may be used for other purposes such a detecting metal fragments such as shrapnel in people. For a battlefield device, a detector head size of 20 x 16 cm gives good coverage, and detects metal objects such as a 9mmm diameter bullet at a range of 30 cm.

In fabrication of the loops on a PCB for example, it is sometimes difficult to get perfect symmetry in the layout so that a small offset signal is obtained when no metal fragment is under the detector. The inventor has determined that the fine balance of the loops to produce nominal zero output can be effected by placing metal "balancing" fragments at suitable position(s) on the PCB itself. This may even be adjustable, such as having two or more screw means 7 (inserted on or through the PCB 8 which carries the loops 1 and 2) on each side of the line of symmetry of the "figure of 8" loop, shown in figure 3.

## Claims

1. A detector to detect metal fragments comprising: a first loop of conducting material, means to feed an rf signal to the first loop, a second loop of conducting material, and means to detect an induced rf signal consequent to said rf signal in said first loop, said second loop being geometrically symmetrically arranged with said first loop such that the rf signal induced is therein is substantially zero when there are no metal fragments in the close proximity of the sensor.

2. A detector as claimed in claim 1 wherein said second loop comprises two halves arranged in anti-phase.

3. A detector as claimed in claim 2 wherein said second loop is in the form of a figure of eight.

4. A detector as claimed in claim s 1 to 3 wherein said loops are arranged to be substantially co-planar.

5. A detector as claimed in claims 1 to 4 wherein said loops are fabricated onto a flat surface.

6. A detector as claimed in claim 1 to 5 wherein said flat surface includes adjustable means to zero output from the second loop.

7. A detector as claimed in any preceding claims having means to determine if the induced signal in the second loop is in phase or quadrature to the signal in the first loop.

8. A detector as claimed in claim 7 which includes means to indicate whether a detected metal fragment is ferromagnetic or non-ferromagnetic.

9. A method of detecting metal fragments in patients comprising using a detector as claimed in any of claims 1 to 8.

10. A method of detecting metal fragments as claimed in claim 9 which includes determining whether any metal fragment is ferromagnetic or non-ferromagnetic.

11. A method as claimed in claims 9 or 10 wherein the rf signal applied to the first coil has a frequency of less than 0.5 MHz.
